# EUROPEAN PATENT SPECIFICATION

(11) **EP 3 177 241 B1**
(45) Date of publication and mention of the grant of the patent: **27.06.2018**
(21) Application number: 15750099.2
(22) Date of filing: 06.08.2015
(51) Int. Cl.: A61F 5/441

(54) **OSTOMY APPLIANCE**
OSTOMIEVORRICHTUNG
ACCESSOIRE DE STOMIE

(30) Priority: 07.08.2014 GB 201414018
(43) Date of publication of application: 14.06.2017
(73) Proprietor: Salts Healthcare Limited, West Midlands B7 4AA (GB)
(72) Inventor: WILTSHIRE, Neil, West Midlands B7 4AA (GB); ARGENT, Peter, West Midlands B7 4AA (GB)
(74) Representative: Purewal, Jagvir Singh
(86) International application number: PCT/GB2015/052276
(87) International publication number: WO 2016/020686

(56) References cited:
- EP-A1- 1 190 688
- WO-A1-03/065945
- WO-A1-2014/076456
- GB-A- 2 270 265

## Description

This invention relates to an ostomy appliance. More particularly, this invention relates to an ostomy appliance including a gas flow regulation device.

An ostomy appliance is used for collecting human waste from a stoma, which is a surgically constructed tube of a user's digestive tract that protrudes through a user's skin. Different types of ostomy appliances are used depending on the type of stoma. If the stoma is from the small intestine (ileum) a drainable ostomy appliance is often preferred, because the waste collected is generally in liquid form and can easily be empted from the appliance. If the stoma is from the large intestine or colon, then a non-drainable ostomy appliance is preferred. This is because the collected waste is usually solid or semi-solid, and it is therefore not possible to easily empty the appliance.

WO2014/076456 discloses an ostomy appliance with a gas vent covered by a cover member which includes an outlet which communicates with the gas vent.

WO03/065945 discloses an ostomy appliance having a wall with one or more vents, covered by a filter with an outlet opening, through which gas may escape. A flap is provided which is sealable to the wall in a manner in which the flap is covering the outlet opening of the filter.

A problem with ostomy appliances, particularly those which are not drainable and thus include a gas outlet (often in combination with a filter), is that 'pancaking' can occur during use. This is where side walls of the bag of the appliance stick together preventing waste from falling to the bottom of the bag. Waste thus collects in the upper region of the bag and can block gas outlets from the bag or even force the bag to become detached from the user.

According to a first aspect of the invention, we provide an ostomy appliance including:-
first and second walls connected to each other at or near their peripheries, the first wall having a stoma-receiving opening; and
a waste collecting cavity defined between the first and second walls,
wherein the second wall includes an outlet which communicates with the cavity, and wherein the appliance includes
a blocking member moveable between a first position in which the outlet is blocked or substantially blocked and a second position in which the outlet is unblocked or substantially unblocked,
characterised in that when said blocking member is in its blocking position the first and second walls are held spaced from each other.

Further features of the invention are set out in claims 2 to 15 appended hereto.

Embodiments of the various aspects of the invention will now be described by way of example only, with reference to the accompanying drawings, of which:
Figure 1 is a front view of an ostomy appliance according to the present invention;
Figure 2 is a plan view of the ostomy appliance of figure 1;
Figure 3 is a further front view of the appliance, but with the blocking member removed;
Figure 4 is a front view of a blocking member; and
Figure 5 is a side view of the blocking member of figure 4.

Referring to the figures, these show an embodiment of an ostomy appliance 10 according to the present invention. In this example the appliance 10 is a non-drainable appliance (although it need not be, so could have a drainable outlet) which has first 12 and second 14 walls connected to each other at or near their peripheries. The walls 12, 14 are made from a suitable plastics material. The first wall 12 has a hydrocolloid member 17 connected thereto with a stoma-receiving opening 16 therein, as is well known in the art. The size of the opening 16 can be increase, typically by cutting, so that it adequately fits a user's stoma.

The appliance 10, in this embodiment includes a further, intermediate, wall 40 positioned in between the first 12 and second 14 walls. The further wall 40 is connected to the first 12 and second 14 walls at or near their peripheries. The further wall 40 and the second wall 14 are connected to each other at a further location 42 other than their peripheries. In this embodiment they are connected by three spaced heat welds 42. There could be fewer or more connections and they need not be heat welds. The purpose of the connections 42 is to hold the wall 40 relative to the second wall 14 when the latter is moved, as will become apparent later.

The wall 40 divides the space between the walls 12, 14 into first 18 and second 43 sub-cavities. The first sub-cavity 18 is the waste collecting cavity and thus communicates with the opening 16 to receive waste.

The further wall 40 includes a passage, in this case an aperture, 41, positioned in an upper region thereof, for flow of gas between the first 18 and second 43 sub-cavities.

The second wall 14 includes an outlet 20 (again, in this example an aperture in the wall) which communicates with the cavity 43, and thus indirectly with the waste cavity 18. The outlet 20 permits gases to ultimately escape from the appliance to atmosphere. In this example the outlet 20 is covered by a gas filter 35 (to remove odours from the escaping gases), but this is not essential.

The further wall 40 is not essential, and could be removed, leaving just a single cavity 18 between the walls 12, 14. In such an embodiment, however, the outlet 20 and its gas filter would have no protection from being contact by waste in the cavity 18, which could lead to blockage of the outlet 20 and ballooning / possible rupture of the appliance. However, alternatives means for preventing blockage to the filter may be provided, for example a hydrophobic covering or cover member.

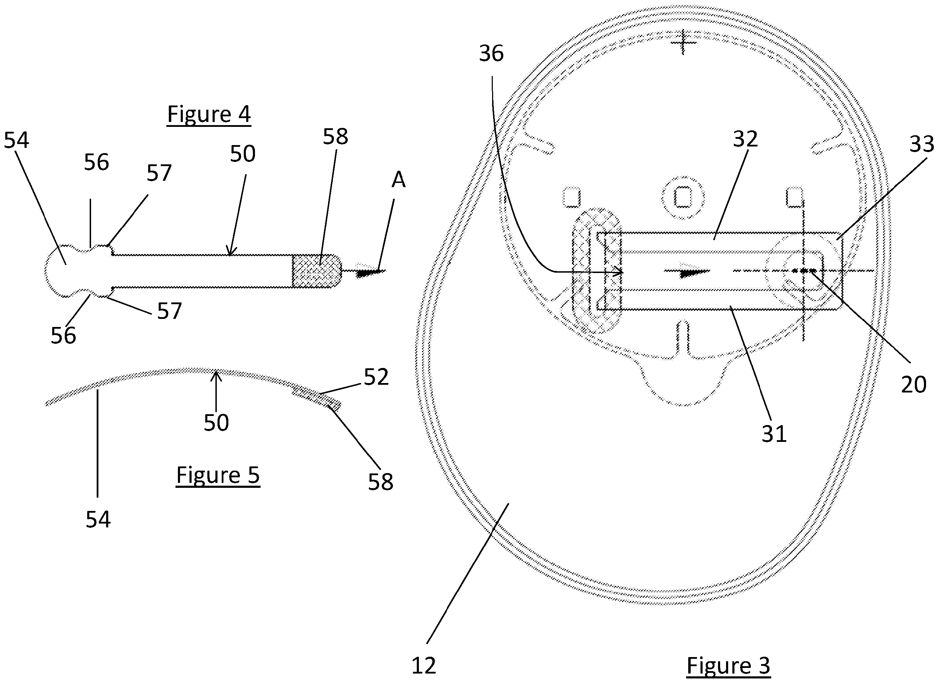

As shown in the figures, the appliance 10 includes a moveable blocking member 50 for blocking or substantially blocking the outlet 20. In this example the blocking member 50 is slidably moveable, but it need not be moveable in that way. The blocking member 50 is an elongate plastics component, which is curved, i.e. non-planar, in side view (see figure 5). The blocking member 50 is made from a resiliently deformable material, such that it can flex. For example, it can be move to a substantially planar condition, but when any bending forces are removed, will revert to its curved position.

The blocking member 50 has a blocking end 52 and a user-graspable end 54, which are opposite each other. The blocking end 54 has connected thereto a deformable, e.g. foam, member 58, which in use faces and directly engages the outlet 20, when in the blocking condition. The member 58 may be resiliently deformable. The end 52 is shaped substantially like the body of a guitar, with opposing recesses 56 for receiving the fingers of a user. A pair of shoulder portions 57 are provided adjacent the recesses 56, the purpose of which will be seen later. Fewer or more recesses 56 may be provided.

The appliance includes a blocking member receiving part 30 which is connected to the second wall 14 by heat welding (although other connection methods could be used). The receiving part 30 could be an integral formation in the wall 14, for example. In this example the receiving part 30 is a substantially rectangular plastics sheet which is heat welded to the wall 14 along sections 31, 32 and 33 to form a U-shaped connection. The U-shaped connection forms a channel or passage 36 which extends laterally across the wall 14 and communicates with the outlet 20 and receives the blocking member 50.

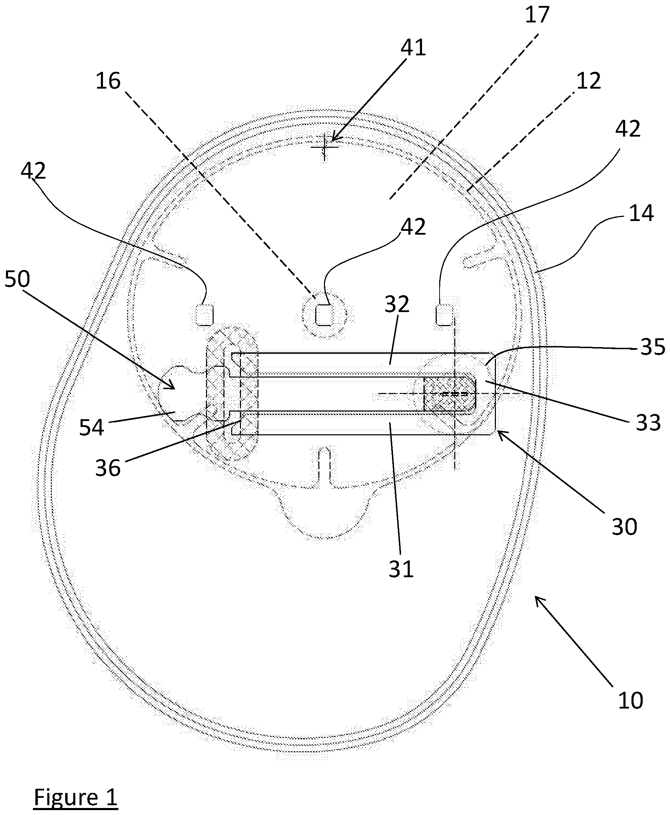

The blocking member 50 is moveable between a first position (see figure 1) in which the outlet 20 is blocked or substantially blocked by the member 58 and a second position (see figure 3) in which the outlet 20 is unblocked or substantially unblocked. In figure 3 the blocking member 50 has been completely removed from the passage 36, so the outlet is completely unblocked. However, it could be slid partially along the passage 36 so that the flow rate of gas exiting the outlet 20 is reduced.

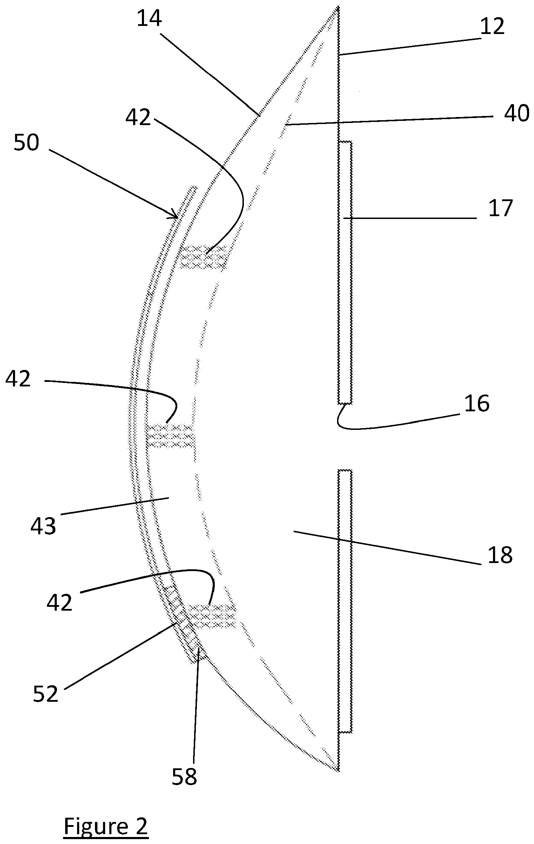

The curved shape of the blocking member 50 means that when the blocking member 50 is in its blocking position (figure 1) the first 12 and second 14 walls are held spaced from each other. As the wall 40 is connected to the second wall 14, the wall 40 is also held spaced from the wall 12. This is illustrated in figure 2, where it can be seen that the second wall 14 and wall 40 adopts a curved or non-planar profile, with the walls 14, 40 curving outwardly away from the first wall 12.

The provision of the blocking member 50 in its blocking position thus holds the walls 14, 40 away from the wall 12 to prevent or at least minimise the occurrence of pancaking. However, the dual functionality of the blocking member 50 means that the user can also control the rate of flow of gas through the outlet, to maintain a desired amount of gas in the cavities 18, 43.

## Claims

1. An ostomy appliance (10) including:-
first and second walls (12, 14) connected to each other at or near their peripheries, the first wall having a stoma-receiving opening (16); and
a waste collecting cavity (18) defined between the first and second walls (12, 14),
wherein the second wall (14) includes an outlet (20) which communicates with the cavity (18), and wherein the appliance (10) includes:
a blocking member (50) moveable between a first position in which the outlet (20) is blocked or substantially blocked and a second position in which the outlet (20) is unblocked or substantially unblocked,
**characterised in that** when said blocking member is in its blocking position the first and second walls are held spaced from each other.

2. An appliance according to any preceding claim wherein the blocking member (50) is slidably moveable between the first and second positions.

3. An appliance according to any preceding claim wherein the blocking member (50) is curved or non-planar.

4. An appliance according to any preceding claim wherein the blocking member (50) is elongate.

5. An appliance according to any preceding claim wherein the blocking member (50) is resiliently deformable.

6. An appliance according to any preceding claim wherein, when the blocking member (50) is in its first position, the or a portion of the second wall (14) adopts a curved or non-planar profile.

7. An appliance according to any preceding claim wherein, when the blocking member (50) is in its first position, the or a portion of the second wall (14) is curved outwardly away from the first wall (12).

8. An appliance according to any preceding claim wherein the blocking member (50) has a blocking end (52) and a user-graspable end (54).

9. An appliance according to claim 8 wherein the user-graspable end (54) is provided with one or more formations.

10. An appliance according to claim 9 wherein the or each formation is a recess (56).

11. An appliance according to any one of claims 8 to 10 wherein the blocking end (52) includes a deformable member (58), preferably wherein the deformable member is resiliently deformable.

12. An appliance according to any preceding claim wherein the second wall (14) includes or has connected thereto a receiving part (30) for receiving the blocking member (50).

13. An appliance according to claim 12 wherein the receiving part (30) includes a channel or passage (36) which communicates with the outlet (20) and/or wherein the receiving part (30) extends laterally across the second wall (14).

14. An appliance according to any preceding claim including a further wall (40) positioned in between the first and second walls (12,14), and dividing the cavity into first and second sub-cavities (18, 43),
preferably wherein the further wall (40) is connected to the first and second walls (12, 14) at or near their peripheries,
even more preferably wherein the further wall (40) includes a passage (41) for flow of gas between the first and second sub-cavities (18, 43), and
most preferably wherein the further wall (40) and the second wall (14) are connected to each other at a further location other than their peripheries.

15. An appliance according to claim 14 wherein when the blocking member (50) is moved to its first position, the second wall (14) and further wall (40) are held spaced from the first wall (12).

## Patentansprüche

1. Stomagerät (10) einschließlich: -
erster und zweiter Wände (12, 14), die miteinander an oder nahe ihrer Peripherien verbunden sind, wobei die erste Wand eine Stoma-Aufnahmeöffnung (16) aufweist; und
eines Abfallsammelhohlraums (18), der zwischen den ersten und zweiten Wänden 12, 14) definiert ist,
wobei die zweite Wand (14) einen Auslass (20) einschließt, der mit dem Hohlaum (18) kommuniziert, und wobei das Gerät (10) einschließt:
ein Blockierungselement (50) aufweist, das zwischen einer ersten Position, in welcher der Auslass (20) blockiert ist oder im Wesentlichen blockiert ist und einer zweiten Position beweglich ist, in welcher der Auslass (20) nicht blockiert oder im Wesentlichen nicht blockiert ist,
**dadurch gekennzeichnet, dass**, wenn sich das Blockierungselement in seiner Blockierungsposition befindet, die ersten und zweiten Wände voneinander beabstandet gehalten werden.

2. Gerät nach einem vorhergehenden Anspruch, wobei das Blockierungselement (50) verschiebbar zwischen den ersten und zweiten Positionen beweglich ist.

3. Gerät nach einem vorhergehenden Anspruch, wobei das Blockierungselement (50) gekrümmt oder nicht ebenflächig ist.

4. Gerät nach einem vorhergehenden Anspruch, wobei das Blockierungselement (50) länglich ist.

5. Gerät nach einem vorhergehenden Anspruch, wobei das Blockierungselement (50) elastisch verformbar ist.

6. Gerät nach einem vorhergehenden Anspruch, wobei, wenn sich das Blockierungselement (50) in seiner ersten Position befindet, der oder ein Abschnitt der zweiten Wand (14) ein gekrümmtes oder nicht ebenflächiges Profil annimmt.

7. Gerät nach einem vorhergehenden Anspruch, wobei, wenn sich das Blockierungselement (50) in seiner ersten Position befindet, der oder ein Abschnitt der zweiten Wand (14) nach außen gerichtet von der ersten Wand (12) weg gekrümmt ist.

8. Gerät nach einem vorhergehenden Anspruch, wobei das Blockierungselement (50) ein Blockierungsende (52) und ein vom Benutzer erfassbares Ende (54) aufweist.

9. Gerät nach Anspruch 8, wobei das vom Benutzer erfassbare Ende (54) mit einer oder mehreren Formationen versehen ist.

10. Gerät nach Anspruch 9, wobei die oder jede Formation eine Vertiefung (56) ist.

11. Gerät nach einem der Ansprüche 8 bis10, wobei das Blockierungsende (52) ein verformbares Element (58) einschließt, vorzugsweise wobei das verformbare Element elastisch verformbar ist.

12. Gerät nach einem vorhergehenden Anspruch, wobei die zweite Wand (14) ein Aufnahmeteil (30) einschließt oder damit verbunden hat, um das Blockierungselement (50) aufzunehmen (50).

13. Gerät nach Anspruch 12, wobei das Aufnahmeteil (30) einen Kanal oder Durchgang (36) einschließt, der mit dem Auslass (20) kommuniziert und/oder wobei sich das Aufnahmeteil (30) lateral über die zweite Wand (14) erstreckt.

14. Gerät nach einem vorhergehenden Anspruch, das eine weitere Wand einschließt, die zwischen den ersten und zweiten Wänden (12, 14) positioniert ist, und den Hohlraum in erste und zweite Unterhohlräume (18, 43) teilt,
vorzugsweise, wobei die weitere Wand (40) mit den ersten und zweiten Wänden (12, 14) an oder nahe ihrer Peripherien verbunden ist,
selbst noch bevorzugter wobei die weitere Wand (40) einen Durchgang (41) für Gasströmung zwischen den ersten und zweiten Unterhohlräumen (18, 43) einschließt, und
am meisten bevorzugt wobei die weitere Wand (40) und die zweite Wand (14) miteinander an einer weiteren Stelle außer ihren Peripherien verbunden sind.

15. Gerät nach Anspruch 14, wobei, wenn das Blockierungselement (50) in seine erste Position bewegt wird, die zweite Wand (14) und die weitere Wand (40) beabstandet von der ersten Wand (12) gehalten werden.

## Revendications

1. Appareillage stomique (10), comprenant :
des première et seconde parois (12, 14) connectées l'une à l'autre au niveau ou à proximité de leurs périphéries, la première paroi ayant une ouverture de réception de stomie (16) ; et
une cavité de collecte de déchets (18) définie entre les première et seconde parois (12, 14),
la seconde paroi (14) comprenant une sortie (20) qui communique avec la cavité (18), et l'appareillage (10) comprenant :
un élément de blocage (50) mobile entre une première position dans laquelle la sortie (20) est bloquée ou sensiblement bloquée, et une seconde position dans laquelle la sortie (20) est débloquée ou sensiblement débloquée,
l'appareillage étant **caractérisé en ce que**, quand ledit élément de blocage est dans sa position de blocage, les première et seconde parois sont maintenues espacées l'une de l'autre.

2. Appareillage selon la revendication précédente, dans lequel l'élément de blocage (50) est mobile coulissant entre les première et seconde positions.

3. Appareillage selon l'une quelconque des revendications précédentes, dans lequel l'élément de blocage (50) est courbé ou non plan.

4. Appareillage selon l'une quelconque des revendications précédentes, dans lequel l'élément de blocage (50) est allongé.

5. Appareillage selon l'une quelconque des revendications précédentes, dans lequel l'élément de blocage (50) est déformable par élasticité.

6. Appareillage selon l'une quelconque des revendications précédentes, dans lequel, quand l'élément de blocage (50) est dans sa première position, la seconde paroi (14) adopte, en partie ou dans sa totalité, un profile courbé ou non plan.

7. Appareillage selon l'une quelconque des revendications précédentes, dans lequel, quand l'élément de blocage (50) est dans sa première position, la seconde paroi (14) est courbée, en partie ou dans sa totalité, vers l'extérieur à l'opposé de la première paroi (12).

8. Appareillage selon l'une quelconque des revendications précédentes, dans lequel l'élément de blocage (50) a une extrémité de blocage (52) et une extrémité saisissable par un utilisateur (54).

9. Appareillage selon la revendication 8, dans lequel l'extrémité saisissable par un utilisateur (54) est munie d'une ou plusieurs formations.

10. Appareillage selon la revendication 9, dans lequel la formation ou chacune des formations est un évidement (56).

11. Appareillage selon l'une quelconque des revendications 8 à 10, dans lequel l'extrémité de blocage (52) comprend un élément déformable (58), l'élément déformable étant de préférence un élément déformable par élasticité.

12. Appareillage selon l'une quelconque des revendications précédentes, dans lequel la seconde paroi (14) comprend ou est connectée à une pièce de réception (30) permettant de recevoir l'élément de blocage (50).

13. Appareillage selon la revendication 12, dans lequel la pièce de réception (30) comprend un canal ou un passage (36) qui communique avec la sortie (20), et/ou dans lequel la pièce de réception (30) s'étend latéralement sur la seconde paroi (14).

14. Appareillage selon l'une quelconque des revendications précédentes, comprenant une autre paroi positionnée entre les première et seconde parois (12, 14), et divisant la cavité entre des première et seconde sous-cavités (18, 43),
l'autre paroi (40) étant de préférence connectée aux première et seconde parois (12, 14) au niveau ou à proximité de leurs périphéries,
l'autre paroi (40) incluant encore plus préférablement un passage (41) pour l'écoulement d'un gaz entre les première et seconde sous-cavités (18, 43), et
idéalement, l'autre paroi (40) et la seconde paroi (14) étant connectées l'une à l'autre au niveau d'un autre emplacement différent de leurs périphéries.

15. Appareillage selon la revendication 14, dans lequel, quand l'élément de blocage (50) est déplacé dans sa première position, la seconde paroi (14) et l'autre paroi (40) sont maintenues espacées de la première paroi (12).
